# EUROPÄISCHE PATENTSCHRIFT

(11) **EP 2 220 017 B1**
(45) Veröffentlichungstag und Bekanntmachung des Hinweises auf die Patenterteilung: **27.04.2011**
(21) Anmeldenummer: 08863473.8
(22) Anmeldetag: 28.10.2008
(51) Int. Cl.: C07C 29/141, C07C 45/50

(54) **MEHRSTUFIGES KONTINUIERLICHES VERFAHREN ZUR HYDROFORMYLIERUNG VON HÖHEREN OLEFINEN ODER OLEFINGEMISCHEN**
MULTISTAGE CONTINUOUS METHOD FOR HYDROFORMYLATION OF HIGHER OLEFINS OR OLEFIN MIXTURES
PROCÉDÉ CONTINU EN PLUSIEURS ÉTAPES POUR L'HYDROFORMYLATION D'OLÉFINES OU DE MÉLANGES OLÉFINIQUES SUPÉRIEURS

(30) Priorität: 20.12.2007 DE 102007061648
(43) Veröffentlichungstag der Anmeldung: 25.08.2010
(73) Patentinhaber: Evonik Oxeno GmbH, 45772 Marl (DE)
(72) Erfinder: LÜKEN, Hans-Gerd, 45770 Marl (DE); KAIZIK, Alfred, 45772 Marl (DE); DREES, Stefan, 48249 Dülmen (DE); BÜSCHKEN, Wilfried, 45721 Haltern Am See (DE)
(86) Internationale Anmeldenummer: PCT/EP2008/064569
(87) Internationale Veröffentlichungsnummer: WO 2009/080395

(56) Entgegenhaltungen:
- DE-A1- 10 241 266
- DE-A1- 19 939 491

## Beschreibung

Die vorliegende Erfindung betrifft ein Verfahren zur kontinuierlichen Herstellung von Aldehyden und/oder Alkoholen durch mehrstufige Hydroformylierung von Olefinen oder Olefingemischen in Gegenwart von unmodifizierten Kobaltkatalysatoren, wobei ein Teil des Kobaltkatalysators von einem Reaktor in einen anderen transportiert wird.

Höhere Alkohole, insbesondere solche mit 6 bis 25 Kohlenstoffatomen, können bekanntlich durch katalytische Hydroformylierung (auch als Oxoreaktion bezeichnet) der um ein Kohlenstoffatom kürzeren Olefine und durch anschließende Hydrierung der gebildeten Aldehyde hergestellt werden. Die Alkohole können als Lösemittel oder als Vorstufe für Detergenzien oder Weichmacher genutzt werden.

Verfahren zur Hydroformylierung von Olefinen sind in der Literatur in großer Zahl beschrieben. Die Wahl des Katalysatorsystems und deroptimalen Reaktionsbedingungen für die Hydroformylierung sind von der Reaktivität des eingesetzten Olefins abhängig. Der Einfluss der Struktur des eingesetzten Olefins auf dessen Reaktivität in einer Hydroformylierungsreaktion ist z. B. von J. FALBE, "New Syntheses with Carbon Monoxide", Springer Verlag, 1980, Berlin, Heidelberg, New York, Seite 95 ff. beschrieben.

Technische Olefingemische, die als Edukte für die Hydroformylierungsreaktion verwendet werden, enthalten oftmals Olefinisomere der verschiedensten Strukturen mit unterschiedlichen Verzweigungsgraden, unterschiedlicher Lage der Doppelbindung und Olefine unterschiedlicher Molmassen. Dies gilt insbesondere für Olefingemische, die durch Di-, Tri-oder weitergehende Oligomerisierung von Olefinen mit 2 bis 8 C-Atomen oder anderen leicht zugänglichen höheren Olefinen bzw. durch Cooligomerisierung der genannten Olefine entstanden sind. Als Beispiele für typische Olefingemische, die technisch für die Hydroformylierung relevant sind, seien Tri- und Tetrapropen sowie Di-, Tri- und Tetrabutene genannt.

Bei einer technisch durchgeführten Hydroformylierung ist es erwünscht, neben einem hohen Umsatz eine hohe Selektivität zu erreichen, um eine optimale Ausnutzung des Rohstoffes zu gewährleisten. Um einen hohen Umsatz zu erreichen, müssen bei langsam reagierenden Olefinen oft eine relativ lange Reaktionszeit und/oder höhere Reaktionstemperaturen in Kauf genommen werden. Reaktivere Olefine werden dagegen unter den gleichen Reaktionsbedingungen schon in weitaus kürzerer Zeit zu den Aldehyden umgesetzt. Bei der gemeinsamen Hydroformylierung von Gemischen von Olefinen unterschiedlicher Reaktivität führt dies dazu, dass man relativ lange Reaktionszeiten benötigt, um einen ausreichenden Umsatz auch der schwerer oxierbaren Olefine zu erzielen. Die aus den leichter umsetzbaren Olefinen entstehenden Aldehyde werden jedoch relativ schnell gebildet und liegen dann neben den schwerer hydroformylierbaren Olefinen im Reaktor vor. Dies führt zu unerwünschten Neben- und Folgereaktionen der Aldehyde, z. B. zur Hydrierung, zu Kondensationsreaktionen sowie zur Bildung von Acetalen und Halbacetalen. Vor allem wegen der unterschiedlichen Reaktivität der Olefinisomeren ist es schwierig, bei einer Hydroformylierungsreaktion hohe Umsätze und gleichzeitig hohe Selektivitäten zu erzielen.

Neben der unvorteilhaften Auswirkung auf die Selektivität gibt es zwei weitere Aspekte, die gegen eine gemeinsame Hydroformylierung von Olefingemischen in einer Stufe bis zu hohen Umsätzen sprechen. Zum einen erfordern die relativ langen Reaktionszeiten bei einer vorgegebenen Kapazität oder Reaktorleistung relativ große Reaktorvolumina. Dies ist insbesondere deshalb nachteilig, weil es sich bei Hydroformylierungsverfahren um Prozesse handelt, die bei erhöhtem Druck ablaufen und die Investitionskosten für Druckreaktoren mit deren Größe exponentiell ansteigen. Zum anderen ist man in der Steuerung des Prozesses in den gewünschten Produkteigenschaften der Aldehyde, z. B. bestimmt durch das Verhältnis der linearen (n) zu verzweigten (i) Aldehyden (n/i-Verhältnis), eingeschränkt.

Als Lösung für die unterschiedlichen Reaktivitäten wurden mehrstufige Reaktionsfahrweisen - mit oder ohne Zwischenabtrennung der in einer Reaktionsstufe gebildeten Produkte - entwickelt.

In GB 1 387 657 wird eine zweistufige Hydroformylierung beschrieben, bei der das Reaktionsprodukt der ersten Stufe gasförmig ausgetragen wird und nach Auskondensation der Aldehyde bzw. Alkohole das Abgas der ersten Stufe, das nicht umgesetzte Olefine enthält, zum einen Teil in die erste Stufe zurückgeführt und zum anderen Teil in einen zweiten Reaktor geleitet wird.

Eine weitere Variante einer zweistufigen Hydroformylierung ist in DE 32 32 557 beschrieben. In der ersten Stufe werden die Olefine unter Verwendung eines Kobaltkatalysators mit Umsätzen von 50 bis 90 % hydroformyliert, der Kobaltkatalysator vom Reaktionsgemisch abgetrennt und die gebildeten Aldehyde zusammen mit den nicht umgesetzten Olefinen in eine zweite Hydroformylierungsstufe eingebracht. Der hier eingesetzte ligandmodifizierte Kobaltkatalysator bewirkt nicht nur die Hydroformylierung der Olefine, sondern gleichzeitig eine Hydrierung der Aldehyde zu den Alkoholen.

In DE 100 34 360 wird ein Verfahren zur mehrstufigen Kobalt- oder Rhodiumkatalysierten Hydroformylierung von Olefinen mit 6 bis 24 Kohlenstoffatomen zu Alkoholen und/oder Aldehyden beschrieben, wobei
a) die Olefine in einem Hydroformylierungsschritt bis zu einem Umsatz von 20 bis 98 % hydroformyliert werden,
b) der Katalysator aus dem so erhaltenen flüssigen Reaktoraustrag entfernt wird,
c) das so erhaltene flüssige Hydroformylierungsgemisch in eine Leichtsiederfraktion, enthaltend Olefine und Paraffine und eine Sumpffraktion, enthaltend Aldehyde und/oder Alkohole getrennt wird,
d) die in der Leichtsiederfraktion enthaltenden Olefine in weiteren Verfahrensstufen, umfassend die Verfahrensschritte a, b und c umgesetzt werden und die Sumpffraktionen der Verfahrensschritte c) aller Verfahrensstufen vereinigt werden.

Bevorzugt wird dieses Verfahren so ausgeübt, dass der flüssige Reaktoraustrag der Hydroformylierungsschritte a) eine homogene Flüssigphase ist. Die Kobalt-oder Rhodium-Katalysatoren werden bevorzugt so eingesetzt, dass sie homogen im flüssigen Reaktoraustrag der Hydroformylierungsschritte a) gelöst sind.

In EP 1 057 803 wird ein zweistufiges Verfahren zur Herstellung von Alkoholen aus Olefinen oder Olefingemischen offenlegt. Dabei wird in der ersten Reaktionsstufe das Einsatzolefin in Gegenwart eines Kobaltkatalysators zu 50 bis 90 % hydroformyliert. Nach der Abtrennung des Katalysators werden vom Reaktionsaustrag die nicht umgesetzten Olefine destillativ abgetrennt und die abgetrennten Olefine im zweiten Hydroformylierungsreaktor umgesetzt. Die Hydroformylierungsprodukte aus beiden Stufen können zu den ensprechenden Alkoholen hydriert werden. In beiden Reaktionsstufen wird als Katalysator Co₂(CO)₈ oder HCo(CO)₄ eingesetzt, der außerhalb der Hydroformylierungsreaktoren erzeugt wird. Aus dem Reaktionsgemisch der Hydroformylierung wird vor Weiterverarbeitung durch Extraktion mit einer Base der Kobaltkatalysator entfernt.

Nachteilig bei diesem Verfahren sind die aufwendige Katalysatoraufarbeitung und die nicht zufriedendstellende Ausbeute. So kann nach Beispiel 5 aus einem Butendimergemisch maximal eine Ausbeute an C₉-Alkoholgemisch von ca. 83 % erhalten werden.

DE 102 41266 beschreibt ein Verfahren zur Hydroformylierung von Olefinen, wobei alle Verfahrensschritte in Gegenwart von Wasser durchgeführt werden.

Ein Hydroformylierungsverrahren, bei dem die Herstellung des aktiven Kobaltkatalysators aus einer wässrigen Kobaltsalzlösung, Extraktion des aktiven Kobaltkatalysators in die organische Phase und Hydroformylierung gleichzeitig in demselben Reaktor erfolgt, wird beispielsweise in DE 196 54 340 beschrieben.

Da die katalytisch wirkenden Kobaltverbindungen (HCo(CO)₄ und Co₂(CO)₈) aus Kobaltsalzen bei Temperaturen unter 160 °C nur langsam gebildet werden, ist ein technisches Verfahren zur Hydroformylierung von Olefinen nach DE 196 54 340 bei Temperaturen unter 160°C oft nicht sinnvoll.

Höhere Temperaturen bei der Hydroformylierung begünstigen jedoch die Bildung von Nebenprodukten, wie beispielsweise die Bildung von Paraffinen durch Hydrierung von Einsatzolefinen. Daher kann es bei einem mehrstufigen Hydroformylierungsverfahren unter Verwendung von unmodifizierten Kobaltkatalysatoren zur Erzielung einer höheren Gesamtausbeute trotzdem vorteilhaft sein, mindestens einen Hydroformylierungsreaktor bei Temperaturen unter 160°C zu betreiben.

Es bestand daher die Aufgabe, ein verbessertes Verfahren zur Herstellung von Oxo-Aldehyden und/oder Alkoholen durch mehrstufige Hydroformylierung von höheren Olefinen mit unmodifizierten Kobaltkomplexen als Katalysator zu entwickeln, bei dem weniger Nebenprodukte bei mindestens gleich bleibender Raum-Zeit-Ausbeute entstehen.

Es wurde nun gefunden, dass bei mehrstufigen kontinuierlichen Hydroformylierungsverfahren, bei denen mindestens zwei Reaktoren mit unmodifizierten Kobaltcarbonyl-Katalysatoren bei unterschiedlichen Temperaturen betrieben werden, die Raum-Zeitausbeute und/oder Selektivität erhöht werden kann, wenn in den Reaktor, in dem die Hydroformylierung bei Temperaturen von über 160 °C nach dem Eintopfverfahren mit gleichzeitiger Katalysatorbildung, Extraktion und Hydroformylierung erfolgt, mit der wässrigen Kobaltsalzlösung mehr Wasser eingebracht wird, als mit dem flüssigen Reaktionsaustrag und dem Synthesegas zusammen ausgetragen wird, und der Überschuss an Wasser durch Abziehen eines Teils der wässrigen Unterphase entfernt wird, und die darin enthaltenden Kobaltcarbonyle in den Reaktor eingebracht werden, der bei niedrigerer Temperatur betrieben wird.

Gegenstand der vorliegenden Erfindung ist demnach ein kontinuierliches Verfahren zur Herstellung von Aldehyden und/oder Alkoholen mit mindestens 6 Kohlenstoffatomen durch mehrstufige Hydroformylierung von Olefinen oder Olefingemischen mit mindestens 5 Kohlenstoffatomen in Gegenwart von unmodifizierten Kobaltkomplexen, wobei mindestens zwei Reaktoren im Temperaturbereich von 100 bis 220 °C und Drücken von 100 bis 400 bar bei unterschiedlichen Temperaturen betrieben werden, das dadurch gekennzeichnet ist,
a) dass ein Reaktor bei Temperaturen von über 160 °C nach dem Eintopfverfahren mit gleichzeitiger Katalysatorbildung, Katalysatorextraktion und Hydroformylierung betrieben wird und in diesen mit der wässrigen Kobaltsalzlösung mehr Wasser in den Reaktor eingespeist wird, als mit dem flüssigen Reaktionsgemisch und mit der Gasphase zusammen aus dem Reaktor getragen wird, wobei zur Konstanthaltung des Stands der wässrigen Kobalt enthaltenden Sumpfphase kontinuierlich ein Teil der wässrigen Sumpfphase aus dem Reaktor gefahren wird,
b) und dass die Kobaltcarbonyle in der abgezogenen wässrigen Phase oder ein Teil davon in den Reaktor, der bei niedrigerer Temperatur betrieben wird, gebracht werden.

Das erfindungsgemäße Verfahren weist im Vergleich zu einem Verfahren, bei denen beide Reaktoren nach dem herkömmlichen Zweistufenprozess betrieben werden, folgende Vorteile auf: Es werden höhere Umsätze von Olefinen zu Hydroformylierungsprodukten erreicht oder bei gleichen Umsatz kann zumindest in einem Reaktor die Reaktionstemperatur gesenkt werden, wodurch die Selektivität für die Bildung von Hydroformylierungsprodukten erhöht wird.

Das erfindungsgemäße Verfahren kann in einer Anlage mit zwei oder mehreren Hydroformylierungsstufen mit unmodifizierten Kobaltcarbonyl-Katalysatoren durchgeführt werden. Dabei werden immer zwei Stufen betrieben, bei denen der erste Hydroformylierungsreaktor bei niedrigerer Temperatur und der zweite Hydroformylierungsreaktor bei der höheren Temperatur betrieben werden. Die Stufe, in der die Umsetzung bei der höheren Temperatur von über 160 °C erfolgt, wird nach dem Eintopfverfahren mit gleichzeitiger Katalysatorbildung, Katalysatorextraktion und Hydroformylierung betrieben. Es können auch beide Hydroformylierungsstufen, die unmodifizierte Kobaltcarbonylkatalysatoren einsetzen, nach dem Eintopfverfahren betrieben werden. Bei Verfahren mit mehr als zwei Hydroformylierungsstufen ist für jede weitere Stufe der Katalysator und die Durchführung frei wählbar.

Das erfindungsgemäße Verfahren kann in mehreren Varianten durchgeführt werden. Der besseren Übersichtlichkeit wegen werden nur zweistufige Verfahren beschrieben.

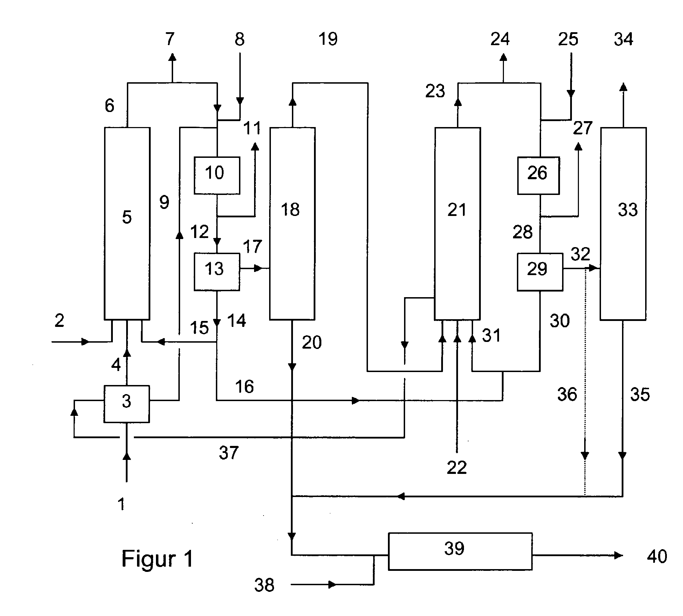

Eine Variante des erfindungsgemäßen Verfahrens ist als Blockschema in Figur 1 wiedergegeben. Das Einsatzolefin (1) extrahiert im Extraktor (3) Kobaltcarbonyle aus Strom (37). Der Extrakt (4), das Synthesegas (2) (Kohlenmonoxid und Wasserstoff) und eine wässrige Kobaltsalzlösung (15) werden in den Hydroformylierungsreaktor (5), der bei niedrigerer Temperatur betrieben wird, eingespeist. Das so erhaltene Hydroformylierungsgemisch (6) wird teilentspannt, das Entspannungsgas (7) (nicht verbrauchtes Synthesegas) wird abgezogen. Die im entspannten Hydroformylierungsgemisch und im wässrigen Strom (9) vorhandenen Kobaltcarbonyle werden zusammen mit einem Sauerstoff enthaltenden Gas (8) im Entkobalter (10) zu Kobaltsalzen oxidiert. Nach Abzug des Abgases (11) wird das Hydroformylierungsgemisch (12) im Behälter (13) in eine praktisch an Kobalt freie organische Phase (17) und in eine wässrige Kobaltsalzlösung (14) getrennt. Ein Teil (15) des Stroms (14) wird in den Hydroformylierungsreaktor (5) zurückgeführt. Der andere Teil (16) wird in den zweiten Hydroformylierungsreaktor (21), der bei der höheren Temperatur betrieben wird, eingespeist. Das katalysatorfreie Hydroformylierungsgemisch (17) wird in der Destillationskolonne (18) in die Leichtsieder (19), die überwiegend aus nicht umgesetzten Olefinen bestehen, und Rohaldehyd (20) getrennt. Die Leichtsieder (19), Synthesegas (22) und Kobaltsalzlösung (31), die durch Vereinigung von Strom (16) mit Strom (30) erhalten wird, werden in den zweiten Hydroformylierungsreaktor (21) gefahren. Aus dem Reaktor (21) wird ein Teil (37) der wässrigen Sumpfphase unter Konstanthaltung deren Stands in den Extraktor (3) geleitet. Das Hydroformylierungsgemisch (23) wird teilentspannt, das Entspannungsgas (24) (nicht verbrauchtes Synthesegas) wird abgezogen. Die im entspannten Hydroformylierungsgemisch (23) vorhandenen Kobaltcarbonyle werden mit Sauerstoff enthaltenden Gas (25) im Entkobalter (26) zu Kobaltsalzen oxidiert. Nach Abzug des Abgases (27) wird das Hydroformylierungsgemisch (28) im Behälter (29) in eine praktisch an Kobalt freie organische Phase (32) und in eine wässrige Kobaltsalzlösung (30), die in den Reaktor (21) zurückgeführt wird, getrennt. Das katalysatorfreie Hydroformylierungsgemisch (32) kann in der Kolonne (33) in die Leichtsieder (34), die überwiegend aus gesättigten Kohlenwasserstoffen bestehen, und Rohaldehyd (35) aufgetrennt werden. Gegebenenfalls kann ein Teil der Leichtsieder (34) in den Reaktor (21) zurückgefahren werden (Leitung in Figur 1 nicht gezeichnet). Eine weitere Ausgestaltung dieser Verfahrensvariante besteht darin, dass das katalysatorfreie Hydroformylierungsgemisch (32) ohne Destillation in der Kolonne (33) (durch Leitung 36) zusammen mit dem Rohaldehyd (20) dem Hydrierreaktor (39) zugeführt wird. Die Rohaldehyde (20) und (35) bzw. (20) und (32) werden im Hydrierreaktor (39) mit Wasserstoff (38) zu den Rohalkoholen (40) hydriert, der optional in einer nicht dargestellten Destillation auf reinen Alkohol aufgearbeitet werden kann. Ist der Aldehyd das eigentliche Zielprodukt, wird die Hydriereinheit (39) umfahren und gegebenenfalls in einer nicht dargestellten Destillation der Rohaldehyd( (20) und (35) oder (20) und (32)) aufgearbeitet.

Alternativ kann auch jeder Aldehydstrom getrennt zu Aldehyden aufgearbeitet oder zu Alkoholen hydriert werden.

Optional kann die Extraktion des Stromes (37) im Extraktor (3) nur mit einer Teilmenge des Einsatzolefin (1) erfolgen.

Eine spezielle Ausführung der Variante 1 ist, die Hydroformylierung im Reaktor (5) praktisch wasserfrei zu betreiben, indem keine wässrige Kobaltsalzlösung (15) in den Reaktor (5) eingespeist wird.

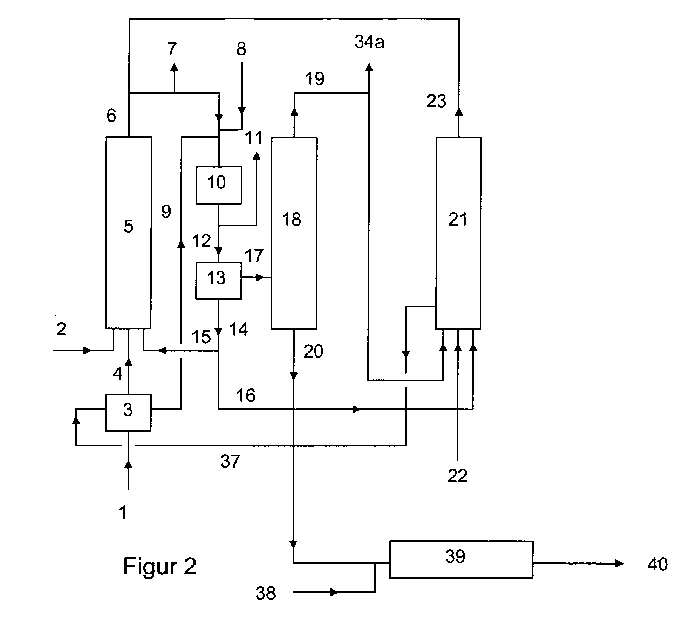

Das Blockschema einer zweiten Variante der Erfindung ist in Figur 2 dargestellt. Das Einsatzolefin (1) extrahiert im Extraktor (3) Kobaltcarbonyle aus Strom (37). Der Extrakt (4), das Synthesegas (2) (Kohlenmonoxid und Wasserstoff) und eine wässrige Kobaltsalzlösung (15) werden in den Hydroformylierungsreaktor (5) eingespeist. Das so erhaltene Hydroformylierungsgemisch (6) wird zusammen mit dem Hydroformylierungsgemisch (23) aus dem zweiten Hydroformylierungsreaktor (21) teilentspannt, das Entspannunggas (7) (nicht verbrauchtes Synthesegas) wird abgezogen. Die im entspannten Hydroformylierungsgemisch und im wässrigen Strom (9) vorhandenen Kobaltcarbonyle werden zusammen mit einem Sauerstoff enthaltenden Gas (8) im Entkobalter (10) zu Kobaltsalzen oxidiert. Nach Abzug des Abgases (11) wird das Hydroformylierungsgemisch (12) im Behälter (13) in eine praktisch an Kobalt freie organische Phase (17) und in eine wässrige Kobaltsalzlösung (14) getrennt. Ein Teil (15) des Stroms (14) wird in den Hydroformylierungsreaktor (5) zurückgeführt. Der andere Teil (16) wird in den zweiten Hydroformylierungsreaktor (21) eingespeist. Das katalysatorfreie Hydroformylierungsgemisch (17) wird in der Destillationskolonne (18) in eine Leichtsiederfraktion (19), die die nicht umgesetzten Olefinen und inerte Paraffine enthält, und Rohaldehyd (20) getrennt. Die Leichtsieder (19) werden, nach Ausschleusung eines Teilstroms (34a) zur Abtrennung von gesättigten Kohlenwasserstoffen (Paraffinen) und sonstigen, nicht olefinischen Verbindungen, zusammen mit Synthesegas (22) und wässrige Kobaltsalzlösung (16) in den zweiten Hydroformylierungsreaktor (21) geleitet. Das entstehende Hydroformylierungsprodukt (23) wird, wie oben beschrieben, zusammen mit dem ersten Hydroformylierungsprodukt (6) aufgearbeitet. Aus dem Reaktor (21) wird ein Teil (37) der wässrigen Sumpfphase unter Konstanthaltung deren Stands in den Extraktor (3) geleitet. Der Rohaldehyd (20) kann in der Hydriereinheit (39) mit Wasserstoff (38) zum Rohalkohol (40) hydriert werden. Dieser Alkohol kann wiederum in einer nicht dargestellten Destillation auf reinen Alkohol aufgearbeitet werden. Wenn ein Aldehyd(gemisch) das Zielprodukt ist, wird der Rohaldehyd (20) unter Umfahrung der Hydriereinheit in einer nicht dargestellten Destillation zu reinem Aldehyd aufgearbeitet werden.

Optional können bei Variante 2 die Ströme (6) und (23) getrennt teilentspannt werden. Ebenso ist möglich, die Kobaltcarbonyle in den Strömen (6) und (23) getrennt zu oxidieren und die oxidierten Ströme zusammen im Behälter (13) zu trennen.

Die Ausschleusung der gesättigten Kohlenwasserstoffe kann anstatt über Strom (34a) auch durch Aufarbeiten eines Teilstroms des vom Katalysator befreiten Hydroformylierungsprodukt (17) erfolgen (nicht dargestellt). Technisch ist dies zum Beispiel durch eine destillative Abtrennung dieses Teilstroms in Leichtsieder, die ausgeschleust werden, und eine Aldehydfraktion, die in die Kolonne (18) eingespeist oder mit dem Rohaldehyd (20) vereinigt wird.

Auch bei Variante 2 kann die Hydroformylierung im Reaktor (5) praktisch wasserfrei durchgeführt werden, indem keine wässrige Kobaltsalzlösung (15) in den Reaktor (5) eingespeist wird.

Bei beiden Varianten können wässrige Kobaltsalzlösungen aus dem Trennbehälter (13) und/oder (29) in den vorgeschalteten Entkobalter (10) und/oder (26) zurückgeführt werden (in den Figuren nicht dargestellt).

Sowohl in Variante 1 als auch in Variante 2 kann die Extraktion der Kobaltcarbonyle aus Strom (37) im Hydroformylierungsreaktor (5), der bei niedrigerer Temperatur als Reaktor (21) betrieben wird, durchgeführt werden (figürlich nicht dargestellt). Das heißt, die aus Reaktor (21) abgezogene wässrige Phase (37) wird direkt in Reaktor (5) eingeleitet. Eine Einspeisung des Kobaltwassers (15) kann dabei entfallen. Es kann in den Reaktor (5) weniger, gleich viel oder mehr Wasser mit der wässrigen Phase (37) eingebracht werden, als das Hydroformylierungsgemisch (6) hinaustragen kann. Im letzteren Falle (in den Figuren nicht dargestellt) wird zur Konstanthaltung des Stands der wässrigen Phase im Reaktor (5) wässrige Phase abgezogen und in den Entkobalter (10) geleitet. Die Extraktion der Kobaltcarbonyle aus Strom (37) im Reaktor (5) ist dann besonders vorteilhaft, wenn Reaktor (5) bei solch niedrigen Temperaturen betrieben wird, bei denen unter Hydroformylierungsbedingungen kaum Kobaltcarbonyle aus Kobalt(II)salzen gebildet werden.

Bei beiden Varianten werden mit den Produkten Wasser und geringe Mengen an Kobaltverbindungen aus dem Prozess ausgetragen. Diese Fehlmengen können periodisch oder kontinuierlich ersetzt werden. Beispielsweise kann eine wässrige Kobaltsalzlösung mit der erforderlichen Konzentration an einer oder mehreren Stellen eingespeist werden. Es kann auch zweckmäßig sein, Kobaltsalzlösungen mit unterschiedlichen Konzentrationen einzusetzen. Bevorzugt werden zum Ausgleich von Verlusten Wasser und Kobaltverbindungen getrennt oder als Lösung in die Behälter (13) und/oder (29) gegeben.

Das gemeinsame Merkmal der Erfindung in den Varianten 1 und 2 ist, dass Kobaltcarbonyle von dem Reaktor, der bei höherer Temperatur betrieben wird, in den Reaktor, in dem die Hydroformylierung bei niedrigerer Temperatur erfolgt, gebracht werden.

Bei der vorliegenden Erfindung wird in den Reaktor, der bei höherer Temperatur betrieben wird, mit der eingespeisten wässrigen Kobaltsalzlösung mehr Wasserphase in den Reaktor gefahren, als mit dem flüssigen Reaktionsgemisch und mit dem überschüssigen Synthesegas zusammen aus dem Reaktor getragen wird. Die relative Wassermenge, die aus dem Reaktor, der bei höherer Temperatur betrieben wird, abgefahren wird, ist insbesondere von der Löslichkeit im Einsatzolefin(gemisch) und dessen Folgeprodukte abhängig. Im Grenzfall, wenn das Hydroformylierungsgemisch praktisch keine Löslichkeit für Wasser aufweist, wird um den Stand der wässrigen Phase im Reaktor konstant zu halten, die gesamte mit der Kobaltsalzlösung eingebrachte Wassermenge aus dem Reaktor gefahren.

Wird Di-n-buten hydroformyliert, so wird so viel wässrige Kobaltsalzlösung in den Reaktor, der bei höherer Temperatur betrieben wird, gefahren, dass zur Konstanthaltung der wässrigen Unterphase bevorzugt 30 bis 90 %, besonders bevorzugt 40 bis 60 % der eingespeisten Wasserphase abgezogen werden müssen.

Die Reaktionstemperatur in diesem Reaktor liegt im Bereich von 160 bis 220 °C, insbesondere im Bereich 175 bis 195 °C.

Die abgezogene wässrige Unterphase wird mit einem Olefin oder Olefingemisch, das in dem Reaktor, der bei tieferer Temperatur betrieben wird, umgesetzt wird, extrahiert. Dabei geht ein Teil der in der wässrigen Phase vorhandenen Kobaltcarbonyle in die Olefinphase über.

Für die Extraktion der Kobaltcarbonyle außerhalb eines Hydroformylierungsreaktor können die dem Fachmann bekannten Extraktionsapparate, wie beispielsweise einfache Extraktionskolonnen, Siebbodenkolonnen, Füllkörperkolonnen oder Kolonnen mit bewegten Einbauten eingesetzt werden. Beispiele für Extraktionsapparate mit bewegten Einbauten sind u. a. der Drehscheibenreaktor und die Scheibelkolonne. Ein weiterer Apparat ist der sogenannte Mischer-Abscheider-Extraktor (mixer-settler-extractor). Es können auch zwei oder mehrere Extraktoren gleicher oder unterschiedlicher Bauart miteinander kombiniert sein.

Dabei ist das Olefin(gemisch) vorzugsweise die disperse Phase. Die Extraktion kann bei der gleichen Temperatur wie in einem vor- oder nachgeschalteten Hydroformylierungsreaktor oder bei tieferen Temperaturen erfolgen. Die Drücke, bei der die Extraktion durchgeführt wird, können die in einem Hydroformylierungsreaktor entsprechen. Die Extraktion kann auch bei einem niedrigeren Druck erfolgen.

Der andere Reaktor des erfindungsgemäßen Verfahrens wird im Temperaturbereich von 120 bis 180 °C, insbesondere im Bereich von 150 bis 175°C betrieben.

Die Hydroformylierung in den beiden Stufen wird jeweils in einem Hochdruckreaktor, bevorzugt Blasensäulenreaktor, durchgeführt. Jede Hydroformylierungsstufe nach dem Eintopfverfahren wird bevorzugt in einem kaskadierten Blasensäulenreaktor durchgeführt, in den Olefine, wässrige Kobaltsalzlösung und Synthesegas bevorzugt mittels einer Mischdüse eingebracht werden.

Der Stand der wässrigen Sumpfphase in dem/den Hydroformylierungsreaktor(en) wird konstant oder nahezu konstant gehalten. Dies bedeutet, dass während eines stationären Betriebs (konstante Reaktionsbedingungen) die Phasengrenzen zwischen unterer wässriger Phase, in der ein Teil der organischen Phase dispergiert ist, einen Stand annimmt, dessen Höhe bevorzugt weniger als ± 5 % um einen Mittelwert schwankt. Dieser Mittelwert der Höhe der Phasengrenze kann unterhalb oder oberhalb oder in Höhe der Austrittsöffnung der Mischdüse liegen, durch die die Edukte in den Reaktor gebracht werden. Die Phasengrenze kann sich dabei 0 bis 1 m, vorzugsweise 0 bis 0,5 m und besonders bevorzugt 0 bis 0,2 m über oder unter der Austrittsöffnung der Mischdüse befinden.

Die Höhe der wässrigen Phase kann sich während einer Laständerung innerhalb der Grenzen des oben genannten Bereichs verschieben. Weiterhin kann sich die Höhe der Wasserphase abhängig vom Durchsatz innerhalb dieser Grenzen verschieben.

Aus dem Reaktor, der bei höherer Temperatur betrieben wird, wird schubweise oder bevorzugt kontinuierlich ein Teil der wässrigen Sumpfphase gefahren.

In dem erfindungsgemäßen Verfahren werden in die Hydroformylierungsreaktoren wässrige Lösungen von Kobaltsalzen eingespeist. Bevorzugt werden wässrige Lösungen von Kobaltsalzen von Carbonsäuren, wie beispielsweise Kobaltformiat oder Kobaltacetat, eingesetzt. Es können auch Lösungen, die mehr als eine Kobaltverbindung enthalten, eingesetzt werden. Eine besonders bevorzugte Kobaltlösung ist diejenige, die bei einer besonders bevorzugten Ausführung des Gesamtprozesses, nämlich bei der oxidativen Entkobaltung des Hydroformylierungsaustrags anfällt. Diese Lösung, die auch Ameisensäure enthält, kann direkt oder nach Aufkonzentrierung oder nach Reduzierung des Ameisensäuregehalts, beispielsweise wie in DE 100 09 207 beschrieben, eingesetzt werden.

Im erfindungsgemäßen Verfahren werden bevorzugt Lösungen eingesetzt, deren Kobaltsalzkonzentration größer als 30 %, insbesondere größer als 60 %, ganz besonders größer als 80 % der Sättigungsgrenze des Kobaltsalzes ist. Ist in der wässrigen Lösung hauptsächlich Kobaltformiat vorhanden, liegt der Gehalt an Kobaltsalzen, berechnet als elementares Kobalt, vorzugsweise im Bereich von 0,7 bis 1,7 Massen-%.

Die Hydroformylierung in den beiden Reaktoren wird vorzugsweise ähnlich ausgeführt, wie in den Patentschriften DE 196 54 340 und DE 101 35 906 dargelegt, mit dem Unterschied, dass aus einem Reaktor ein Teil der wässrigen Kobaltverbindungen enthaltenden Unterphase abgetrennt wird.

Der Reaktionsdruck liegt im Bereich von 100 bis 400 bar, insbesondere im Bereich von 150 bis 300 bar. Im eingesetzten Synthesegas liegt das Volumenverhältnis von Wasserstoff zu Kohlenstoffmonoxid im Bereich 1 zu 2 bis 2 zu 1.

In der vorliegenden Erfindung werden das Olefin(gemisch), die wässrige Lösung mit den Kobaltverbindungen und Synthesegas (Gemisch aus Wasserstoff und Kohlenstoffmonoxid) und gegebenenfalls ein Lösemittel in den Sumpf mindestens eines Hydroformylierungsreaktors eingebracht. Im Sumpf des Reaktors, der zur Erzeugung des aktiven Katalysators bei Temperaturen über 160 °C betrieben wird, liegt eine wässrige Phase vor, in der geringe Mengen an organischer Phase dispergiert ist. Die wässrige Phase beträgt 5 bis 30 %, insbesondere 10 bis 30 % des flüssigen Reaktorinhalts. In dem anderen Reaktor, in den aktiver Kobaltkatalysator eingebracht wird, beträgt die wässrige Sumpfphase 0 bis 20 %, insbesondere 5 bis 15 % des flüssigen Reaktorinhalts.

Um eine hohe Reaktionsgeschwindigkeit zu erhalten, ist es zweckmäßig, die wässrige Sumpfphase mit der organischen Phase und Synthesegas sowie die wässrige Phase zu vermischen. Durch die intensive Vermischung werden Konzentrationsgradienten der Reaktionspartner vermieden. Darüber hinaus begünstigt die Vermischung der wässrigen Sumpfphase mit der organischen Phase den Übergang des gebildeten Katalysators in die organische Phase, in der die Hydroformylierung hauptsächlich abläuft.

Die Vermischung der Reaktionskomponenten (Olefin, Synthesegas, wässrige Kobaltsalzlösung) mit sich selbst und/oder Hydroformylierungsgemisch sowie die Vermischung der beiden flüssigen Phasen im Reaktor kann mit Hilfe geeigneter technischer Einrichtungen erfolgen.

Olefin, Synthesegas und wässrige Kobaltsalzlösung können getrennt, zweckmäßig durch Düsen, in den Reaktor eingeleitet werden. Es können auch zwei Komponenten zusammen durch eine oder mehrere Mischdüsen und die dritte Komponente getrennt in den Reaktor eingespeist werden. Zweckmäßig ist es jedoch, alle drei Komponenten zusammen durch eine oder mehrere Mischdüsen dem Reaktor zuzuführen.

Die wässrige Sumpfphase kann mit Hilfe einer Pumpe, die in einer Umlaufleitung installiert ist, umgewälzt werden. Eine Durchmischung der wässrigen Phase und Vermischung der wässrigen Phase mit der organischen Phase und Synthesegas kann auch dadurch erreicht werden, dass ein Teil der wässrigen Phase aus dem Reaktor der Mischdüse für die Einsatzstoffe zugeleitet wird. Dies kann mit Hilfe einer Pumpe geschehen.

Die Ejektorwirkung von Mischdüsen wird durch den Impuls des austretenden Gases und der austretenden Flüssigkeit beeinflusst. Hohe Flüssigkeitsgeschwindigkeiten von 3 bis 300 m/s, besonders 10 bis 100 m/s, ganz besonders von 15 bis 70 m/s am Ort beziehungsweise an den Orten der Einmischung werden bevorzugt.

Das Reaktionsgemisch aus einem Hydroformylierungsreaktor enthält Einsatzstoff (Olefine), Produkte (Aldehyde, Alkohole, Ameisensäureester), Nebenprodukte und Kobaltcarbonylverbindungen. Die letzteren können aus dem Reaktionsgemisch nach an sich bekannten technischen Verfahren abgetrennt werden. Bevorzugt erfolgt die Abtrennung der Kobaltcarbonyle oxidativ. Dazu wird das Reaktionsgemisch teilentspannt, insbesondere auf 10 bis 15 bar, und in Gegenwart von einer sauren Kobalt(II)salzlösung mit Sauerstoff enthaltenen Gasen, insbesondere Luft oder Sauerstoff, bei Temperaturen von 90 °C bis 160 °C in einem Reaktor (Entkobalter) umgesetzt und so oxidativ von Kobalt-Carbonylverbindungen befreit. Diese werden dabei unter Bildung von Kobalt(II)salzen zerstört. Die Entkobaltungsverfahren sind gut bekannt und in der Literatur ausführlich, wie z. B. in "New Syntheses with Carbon Monoxide", Springer Verlag (1980), Berlin, Heidelberg, New York, Seite 158 f., beschrieben. Nach der Oxidation wird das Gemisch in die organische Produktphase, Abgas und Prozesswasser getrennt. Das abgetrennte Prozesswasser weist einen pH-Wert von 1,5 bis 4,5 und einen Kobaltgehalt zwischen 0,5 und 2 Massen-% auf. Der größte Teil des Prozesswassers wird gegebenenfalls unter Zusatz von Wasser in den Entkobalter zurückgefahren. Der andere Teil wird vorzugsweise in den Hydroformylierungsreaktor zurückgefahren.

Die nach der Abtrennung der Kobaltcarbonyle enthaltenen organischen Reaktionsgemische werden nach bekannten Verfahren aufgearbeitet. Beispielsweise können sie durch Destillation in Kohlenwasserstofffraktionen, die nicht umgesetzte Olefine enthalten kann, in Aldehyde, andere Wertprodukte (Alkohole und deren Formiate) und sonstige Stoffe getrennt werden. Die Kohlenwasserstofffraktionen mit nicht umgesetzten Olefinen können teilweise in die gleiche erfindungsgemäße Hydroformylierung zurückgeführt werden oder in einer weiteren Hydroformylierung, die auch erfindungsgemäß betrieben werden kann, umgesetzt werden. Die gewonnenen Aldehyde können als solche verwendet werden oder können Ausgangsstoff für die Herstellung anderer Stoffe sein, beispielsweise Carbonsäuren, Amine, Nitrile oder Aldolkondensationsprodukte.

Weiterhin können die Hydroformylierungsgemische vor oder nach Abtrennung der nicht umgesetzten Olefine zu den entsprechenden primären Alkoholen hydriert werden, die u. a. als Vorstufen für Weichmacher oder Detergenzien eingesetzt werden können.

Als Edukte für das erfindungsgemäße Verfahren können in Prinzip alle Olefine mit mindestens 5 Kohlenstoffatomen eingesetzt werden. Die eingesetzten Edukte können lineare oder verzweigte α-Olefine, lineare oder verzweigte Olefine mit innenständigen Doppelbindungen, cycloaliphatische Olefine oder Olefine mit aromatischen Gruppen sein. Es können Stoffe mit einer oder mehreren olefinischen Doppelbindung(en) eingesetzt werden. Vorzugsweise werden Olefine oder Olefingemische mit 6 bis 24 Kohlenstoffatomen eingesetzt. Die Gemische können aus Olefinen gleicher, ähnlicher oder deutlich unterschiedlicher C-Zahl bestehen. Als Olefine, die entweder in reiner Form, in einem Isomerengemisch oder in einem Gemisch mit weiteren Olefinen anderer C-Zahl als Edukt eingesetzt werden können, seien beispielsweise genannt: 1-, 2- oder 3-Hexen, 1-Hepten, lineare Heptene mit innenständiger Doppelbindung (2-Hepten, 3-Hepten usw.), Gemische linearer Heptene, 2- oder 3-Methyl-1-hexen, 1-Octen, lineare Octene mit innenständiger Doppelbindung, Gemische linearer Octene, 2- oder 3-Methylhepten, 1-Nonen, lineare Nonene mit innenständiger Doppelbindung, Gemische linearer Nonene, 2-, 3- oder 4-Methyloctene, 1-, 2-, 3-, 4- oder 5-Decen, 2-Ethyl-1-octen, 1-Dodecen, lineare Dodecene mit innenständiger Doppelbindung, Gemische linearer Dodecene, 1-Tetradecen, lineare Tetradecene mit innenständiger Doppelbindung, Gemische linearer Tetradecene, 1-Hexadecen, lineare Hexadecene mit innenständiger Doppelbindung, Gemische linearer Hexadecene. Geeignete Edukte sind weiterhin u. a. das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende Hexadecen-Gemisch (Tetrabuten) sowie durch Cooligomerisierung von Olefinen mit unterschiedlicher C-Zahl hergestellte Olefingemische, gegebenenfalls nach destillativer Trennung in Fraktionen mit gleicher oder ähnlicher C-Zahl. Weiterhin können Olefine oder Olefingemische, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden. Bevorzugte Edukte sind Gemische isomerer Octene-, Nonene-, Dodecene- oder Hexadecene, d. h. Oligomere von niedrigen Olefinen, wie n-Butenen, Isobuten oder Propen. Andere ebenfalls gut geeignete Edukte sind Oligomere aus C₅-Olefinen.

Wenn C₈-, C₁₂- oder C₁₆- Olefingemische die Edukte sind, werden insbesondere solche eingesetzt, die durch Oligomerisierung von linearen Butenen an NickelFestbettkatalysatoren hergestellt worden sind, wie beispielsweise nach dem Octol-Prozess (Hydrocarbon Process, Int. Ed. (1986) 65 (2.Sect. 1) Seite 31 - 33).

Die Hydroformylierungsgemische können zur Herstellung von Aldehyden verwendet werden. Aus den Hydroformylierunggemischen können die entsprechenden Alkohole hergestellt werden, die beispielsweise als Vorstufe für Weichmacher, Detergenzien oder Schmiermittel verwendet werden. Durch Oxidation können aus den Hydroformylierungsgemischen die entsprechenden Carbonsäuren hergestellt werden, die beispielsweise als Vorstufe für Lackadditive oder Vinylester eingesetzt werden.

Die folgenden Beispiele sollen die Erfindung erläutern, ohne diese darauf zu beschränken.

### Beispiel 1 Herstellung von aktivem Kobaltkatalysator durch Vorcarbonylierung

1 a) Carbonylierung bei 190°C
   In einem 2 I Rührautoklaven aus Edelstahl wurden 1000 ml wässrige Kobaltacetatlösung mit 1,2 Massen % Kobalt gerechnet als Metall vorgelegt. Unter intensiven Rühren (1000 Upm) wurde in den Hochdruckautoklaven Synthesegas mit einem CO/H₂-Volumenverhältnis von 1 zu 1 bei 190 °C und 280 bar eingeleitet. Durch zeitlich versetzte Probenahme konnte die Bildung der Kobaltcarbonylkomplexe während der Carbonylierung analytisch verfolgt werden. Nach einer Carbonylierungszeit von 4 Stunden waren rd. 66 % des eingesetzten Kobaltsalzes zu dem aktiven Kobaltkatalysator umgesetzt.
1b) Der Versuch 1a) wurde wiederholt mit dem Unterschied, dass anstelle der wässrigen Kobaltacetat-Lösung eine wässrige Kobaltacetat/Isononanol-Gemisch (200 ml Isononanol und 800 ml Wasser mit 1,2 Massen-% Kobalt, berechnet als Metall und bezogen auf die Gesamtlösung) eingesetzt wurde. Bereits nach 5 Minuten bei 190 °C waren 65 % des eingesetzten Kobalts zu Kobaltcarbonylkomplexen umgesetzt.
1c) Carbonylierung bei 160 °C
   In einem dritten Versuch wurde der Einfluss der Temperatur auf die Bildung des aktiven Kobaltkatalysators untersucht. Dabei wurde wie bei Versuch 1a) vorgegangen, mit dem einzigen Unterschied, dass die Reaktionstemperatur 160 °C betrug. Nach einer Carbonylierungszeit von 4 Stunden waren bei 160 °C lediglich rd. 25 % des eingesetzten Kobaltsalzes zu dem aktiven Kobaltkatalysator umgesetzt.
1d)
   Die Wiederholung des Versuches 1c) mit dem gleichen Einsatzgemisch wie bei Versuch 1 b ergab, dass ein 25%-iger Umsatz des eingesetzten Kobaltsalzes zu den Kobaltcarbonylkomplexen nach rd. 50 Minuten Versuchszeit erzielt worden war.
   Der Vergleich der Versuche 1a und 1c sowie der Versuche 1b und 1d zeigt den deutlichen Temperatureffekt. Bei 160 °C ist selbst bei Zusatz von Isononanol die Bildung der Kobaltcarbonyle für einen technischen Prozess zu langsam.

### Beispiel 2 Vergleichbeispiel

### Nonanole durch zweistufige Hydroformylierung von Dibuten

### 1. Stufe

In einem 5l Hochdruckautoklaven mit Rührer und elektrischer Heizung wurden 2000 g Di-buten (15,2 Massen % n-Octene, 61,9 Massen % 3-Methylheptene, 22,9 Massen % 3,4-Dimethylhexene) in Gegenwart eines Kobaltkatalysators bei 180°C und einem Synthesegasdruck von 280 bar 2 Stunden lang hydroformyliert. Der aktive Kobalt-Katalysator war gemäß Beispiel 1 a hergestellt worden, indem 640 g einer wässrigen Kobaltacetat-Lösung mit 1,2 Massen-% Kobalt 4 Stunden bei 190°C und 280 bar mit Synthesegas behandelt wurden. Nach Abkühlen und Entspannen wurden die gebildeten Kobaltcarbonyle durch Extraktion mit den 2000 g Dibuten in die organische Phase überführt. Die Konzentration des aktiven Katalysators im Dibuten betrug 0,040 Massen-% bezogen auf Dibuten und gerechnet als Kobaltmetall.

Nach Abkühlen auf 80°C und Entspannung wurde das Hydroformylierungsgemisch durch Behandlung mit 5 Massen-%iger wässriger Essigsäure in Gegenwart von Luft von Kobalt befreit. Das entkobaltete Hydroformylierungsgemisch wurde anschließend von der wässrigen Phase abgetrennt.

Die Hydroformylierung wurde unter gleichen Bedingungen fünfmal wiederholt.

Die entkobalteten Hydroformylierungsgemische wurden vereinigt. Es wurden 11950 g Hydroformylierungsgemisch erhalten. Die Zusammensetzung des Produktgemisches gemäß GC-Analyse ist in Tabelle 1, Spalte 2 angegeben. Danach betrug der Dibuten-Umsatz 82,7 % und die Wertprodukt-Selektivität 89,6 %, entsprechend einer Wertproduktausbeute von 74,1 %. Als Wertprodukte wurden hier Nonanale, Nonanole und deren Formiate betrachtet.

### 2. Stufe

10500 g entkobaltetes Hydroformylierungsgemisch aus der ersten Stufe wurden zur Rückgewinnung von nicht umgesetzten Olefinen in einer Kolonne destilliert. Die C₈-Kohhlenwasserstoffe (Olefine und Paraffine) wurden als Leichtsieder am Kolonnenkopf abgezogen, der Kolonnensumpf enthielt die Wertprodukte und die Hochsieder.

2000 g des rückgewonnenen C₈-Kohlenwasserstoffgemisches mit rd. 75,1 % C₈-Olefinen und rd. 24,9 % Paraffinen wurden in dem 51 Autoklaven bei 185°C und einem Synthesegasdruck von 280 bar 3 Stunden lang hydroformyliert. Der aktive Kobaltkatalysator war wie in der 1. Stufe hergestellt und in die Olefinphase überführt worden, seine Konzentration betrug 0,040 Massen-% Kobalt bezogen auf das Olefin und gerechnet als Kobaltmetall.

Das Hydroformylierungsgemisch wurde auf 80 °C abgekühlt, entspannt und entkobaltet, wie in der 1. Stufe beschrieben. Man erhielt 2366 g entkobaltetes Hydroformylierungsgemisch, dessen Zusammensetzung gemäß GC-Analyse in Tabelle 1, Spalte 3 wiedergegeben ist. Der Olefinumsatz betrug 91 % und die Wertprodukt-Selektivität 83,8 %, entsprechend einer Wertproduktausbeute von 76,3%.

Der Gesamtolefinumsatz über beide Stufen betrug 98,4 % bei einer Wertprodukt-Selektivität von 88,6 %, entsprechend einer Gesamtwert-Produktausbeute von 87,3 % bezogen auf eingesetztes Dibuten.

### Beispiel 3

### Nonanole durch zweistufige Hydroformylierung von Dibuten (erfindungsgemäß)

### 1. Stufe

In dem in Beispiel 1 verwendeten 5l Hochdruckautoklaven werden 2000 g Dibuten in Gegenwart eines aktiven Kobaltkatalysators bei 165°C und einem Synthesegasdruck von 280 bar 4 Stunden lang hydroformyliert. Der aktive Kobalt-Katalysator war wie in Beispiel 2 hergestellt und in Dibuten extrahiert worden. Die Konzentration des Katalysators im Dibuten betrug 0,040 Massen-%, bezogen auf Dibuten und gerechnet als Kobaltmetall.

Nach Abkühlen auf 80°C wurde das Hydroformylierungsgemisch entspannt und durch Behandlung mit 5 Massen-%iger wässriger Essigsäure und Luft von Kobalt befreit.

Das Hydroformylierung wurde unter gleichen Bedingungen fünfmal durchgeführt. Die entkobalteten Hydroformylierungsgemische wurden vereinigt. Es werden 11750 g Hydroformylierungsgemisch erhalten; die Zusammensetzung gemäß GC-Analyse ist in Tabelle 2, Spalte 2 angegeben. Danach betrug der Dibuten-Umsatz 72,4 % und die Wertprodukt-Selektivität 94,2 %, entsprechend einer Wertproduktausbeute von 68,2 %. Als Wertprodukte wurden hier Nonanale, Nonanole und deren Formiate betrachtet.

Man erkennt, dass die Wertprodukt-Selektivität bei Hydroformylierung bei 165°C im Vergleich zur Hydroformylierung bei 180°C, wie in der ersten Stufe des Beispiels 2 ausgeführt, deutlich gesteigert wurde.

### 2. Stufe

10000 g entkobaltetes Hydroformylierungsgemisch aus der ersten Stufe wurden wie im Beispiel 2 zwecks Rückgewinnung der nicht umgesetzten Olefinen in einer Kolonne destilliert. Die C₈-Olefine und C₈-Paraffine wurden als Kopffraktion abgezogen, der Kolonnensumpf enthielt die Wertprodukte und die Hochsieder.

2000 g rückgewonnenes C₈-Kohlenwasserstoffgemisch (95,1 C₈-Olefine und 4,9 % Paraffine) werden in dem 51 Autoklaven der 1. Stufe bei 185°C und einem Synthesegasdruck von 280 bar 5 Stunden lang hydroformyliert. Der Kobaltkatalysator war wie in Beispiel 2 hergestellt und in die Olefinphase überführt worden, seine Konzentration betrug 0,045 Massen-% bezogen auf das Olefin und gerechnet als Kobaltmetall.

Das Hydroformylierungsgemisch wurde auf 80 °C abgekühlt, entspannt und entkobaltet, wie in der 1. Stufe beschrieben. Man erhielt 2465 g entkobaltetes Hydroformylierungsgemisch, dessen Zusammensetzung gemäß GC-Analyse in Tabelle 2, Spalte 3 wiedergegeben ist. Der Olefinumsatz betrug 91,8 % und die Wertprodukt-Selektivität 84,8 %, entsprechend einer Wertproduktausbeute von 77,9%.

Der Gesamtolefinumsatz über beide Stufen betrug 97,7 % bei einer Wertprodukt-Selektivität von 91,8 %, entsprechend einer Gesamtwert-Produktausbeute von 89,7 % bezogen auf eingesetztes Dibuten.

**Tabelle 1 : Zusammensetzung von entkobalteten Hydroformylierungsausträgen Beispiel 2**

| | Bsp 2, 1. Stufe Massen-% | Bsp. 2, 2. Stufe Massen-% |
|---|---|---|
| C₈-Olefine | 14,5 | 5,7 |
| C₈-Paraffine | 4,8 | 24,5 |
| C₉-Aldehyde | 58,8 | 38,3 |
| Nonylformiate | 3,5 | 4,8 |
| C₉-Alkohole | 17,1 | 19,4 |
| Hochsieder | 1,3 | 7,3 |

**Tabelle 2: Zusammensetzung von entkobalteten Hydroformylierungsausträgen Beispiel 3**

| | Bsp 3, 1. Stufe Massen-% | Bsp. 3, 2. Stufe Massen-% |
|---|---|---|
| C₈-Olefine | 23,5 | 6,3 |
| C₈-Paraffine | 1,2 | 8,6 |
| C₉-Aldehyde | 59,8 | 43,0 |
| Nonylformiate | 3,2 | 5,6 |
| C₉-Alkohole | 11,3 | 29,0 |
| Hochsieder | 1,0 | 7,5 |

Somit liefert das erfindungsgemäße Verfahren, wie der Vergleich von Beispiel 3 mit Beispiel 2 zeigt, eine um 2,4 % höhere Produktausbeute.

## Patentansprüche

1. Verfahren zur kontinuierlicher Herstellung von Aldehyden und/oder Alkoholen mit mindestens 6 Kohlenstoffatomen durch mehrstufige Hydroformylierung von Olefinen oder Olefingemischen mit mindestens 5 Kohlenstoffatomen in Gegenwart von unmodifizierten Kobaltkomplexen, wobei mindestens zwei Reaktoren im Temperaturbereich von 100 bis 220 °C und Drücken von 100 bis 400 bar bei unterschiedlichen Temperaturen betrieben werden,
**dadurch gekennzeichnet,**
a) **dass** ein Reaktor bei Temperaturen über 160 °C nach dem Eintopfverfahren mit gleichzeitiger Katalysatorbildung, Katalysatorextraktion und Hydroformylierung betrieben wird und in diesen mit der wässrigen Kobaltsalzlösung mehr Wasser in den Reaktor gespeist wird, als mit dem flüssigen Reaktionsgemisch und mit der Gasphase zusammen aus dem Reaktor getragen wird, wobei zur Konstanthaltung des Stands der wässrigen Sumpfphase ein Teil der wässrigen Sumpfphase aus dem Reaktor gefahren wird,
b) und **dass** die Kobaltcarbonyle in der abgezogenen wässrigen Phase oder ein Teil davon in den Reaktor, der bei niedrigerer Temperatur betrieben wird, gebracht werden.

2. Verfahren nach Anspruch 1,
**dadurch gekennzeichnet,**
**dass** Kobaltcarbonyle in der abgezogenen wässrigen Phase mit einem Olefin oder Olefingemisch extrahiert werden und dass der Olefinextrakt mit Kobaltcarbonylen in den Reaktor gefahren wird, der bei niedrigerer Temperatur unter Verwendung von unmodifizierten Kobaltkatalysatoren betrieben wird.

3. Verfahren nach Anspruch 1,
**dadurch gekennzeichnet,**
**dass** die abgezogene wässrige Phase in den Reaktor, der bei niedrigerer Temperatur unter Verwendung von unmodifizierten Kobaltkatalysatoren betrieben wird, eingeleitet wird.

4. Verfahren nach den Ansprüchen 1 bis 3,
**dadurch gekennzeichnet,**
**dass** die Hydroformylierung zweistufig erfolgt.

5. Verfahren nach Anspruch 4,
**dadurch gekennzeichnet,**
**dass** die Reaktionstemperatur in dem Reaktor, der bei höherer Temperatur betrieben wird, im Bereich von 160 bis 220 °C und die Reaktionstemperatur in dem Reaktor, der bei niedrigerer Temperatur betrieben wird, im Bereich von 120 bis 180 °C liegt.

6. Verfahren nach Anspruch 4,
**dadurch gekennzeichnet,**
**dass** die Reaktionstemperatur in dem Reaktor, der bei höherer Temperatur betrieben wird, im Bereich von 175 bis 195 °C und die Reaktionstemperatur in dem Reaktor, der bei niedrigerer Temperatur betrieben wird, im Bereich von 150 bis 175°C liegt.

7. Verfahren nach den Ansprüchen 1 bis 6,
**dadurch gekennzeichnet,**
**dass** Olefine oder Olefingemische mit 6 bis 24 C-Atomen hydroformyliert werden.

8. Verfahren nach Anspruch 7,
**dadurch gekennzeichnet,**
**dass** Olefine oder Olefingemische mit 8 bis 16 C-Atomen hydroformyliert werden.

9. Verfahren nach Anspruch 7 oder 8,
**dadurch gekennzeichnet,**
**dass** Butenoligomere hydroformyliert werden.

10. Verfahren nach Anspruch 9,
**dadurch gekennzeichnet,**
**dass** Oligomere, hergestellt aus linearen Butenen unter Verwendung von Nickelfestbettkatalysatoren, hydroformyliert werden.

## Claims

1. Process for the continuous preparation of an aldehyde and/or alcohol having at least 6 carbon atoms by multistage hydroformylation of an olefin or olefin mixture having at least 5 carbon atoms in the presence of unmodified cobalt complexes, in which at least two reactors are operated at different temperatures in the temperature range from 100 to 220°C and pressures of from 100 to 400 bar, **characterized in that**
a) one reactor is operated at temperatures above 160°C by the one-pot process with simultaneous catalyst formation, catalyst extraction and hydroformylation and the amount of water fed into the reactor with the aqueous cobalt salt solution is greater than that discharged from the reactor with the liquid reaction mixture and the gas phase together, with part of the aqueous bottom phase being discharged from the reactor to keep the level of the aqueous bottom phase constant,
b) and the cobalt carbonyls in the aqueous phase taken off or part thereof are introduced into the reactor which is operated at a lower temperature.

2. Process according to Claim 1,
**characterized in that** cobalt carbonyls in the aqueous phase taken off are extracted by means of an olefin or olefin mixture and the olefin extract containing cobalt carbonyls is fed into the reactor which is operated at a relatively low temperature using unmodified cobalt catalysts.

3. Process according to Claim 1,
**characterized in that** the aqueous phase taken off is introduced into the reactor which is operated at a relatively low temperature using unmodified cobalt catalysts.

4. Process according to any of Claims 1 to 3,
**characterized in that** the hydroformylation is carried out in two stages.

5. Process according to Claim 4,
**characterized in that** the reaction temperature in the reactor which is operated at a relatively high temperature is in the range from 160 to 220°C and the reaction temperature in the reactor which is operated at a lower temperature is in the range from 120 to 180°C.

6. Process according to Claim 4,
**characterized in that** the reaction temperature in the reactor which is operated at a relatively high temperature is in the range from 175 to 195°C and the reaction temperature in the reactor which is operated at a lower temperature is in the range from 150 to 175°C.

7. Process according to any of Claims 1 to 6,
**characterized in that** an olefin or olefin mixture having from 6 to 24 carbon atoms is hydroformylated.

8. Process according to Claim 7,
**characterized in that** an olefin or olefin mixture having from 8 to 16 carbon atoms is hydroformylated.

9. Process according to Claim 7 or 8,
**characterized in that** a butane oligomer is hydroformylated.

10. Process according to Claim 9,
**characterized in that** an oligomer prepared from linear butenes using fixed- bed nickel catalysts is hydroformylated.

## Revendications

1. Procédé pour la préparation en continu d'aldéhydes et/ou d'alcools comprenant au moins 6 atomes de carbone par hydroformylation en plusieurs étapes d'oléfines ou de mélanges d'oléfines comprenant au moins 5 atomes de carbone en présence de complexes de cobalt non modifiés, où au moins deux réacteurs sont exploités dans la plage de température de 100 à 220°C et à des pressions de 100 à 400 bars à des températures différentes, **caractérisé**
a) **en ce qu'**un réacteur est exploité à des températures supérieures à 160°C selon le procédé dans un pot avec une formation de catalyseur, extraction du catalyseur et hydroformylation simultanées et on injecte dans ce réacteur plus d'eau avec la solution aqueuse de sel de cobalt que la quantité évacuée du réacteur avec le mélange réactionnel liquide et la phase aqueuse ensemble, en évacuant une partie de la phase aqueuse de fond du réacteur en vue de maintenir le niveau de la phase aqueuse de fond constant,
b) et **en ce que** les cobalt-carbonyles dans la phase aqueuse soutirée ou une partie de ceux-ci sont amenés dans le réacteur qui est exploité à température plus basse.

2. Procédé selon la revendication 1, **caractérisé en ce que** les cobalt-carbonyles dans la phase aqueuse soutirée sont extraits avec une oléfine ou un mélange d'oléfines et **en ce que** l'extrait d'oléfines avec les cobalt-carbonyles est guidé dans le réacteur qui est exploité à température plus basse avec utilisation de catalyseurs au cobalt non modifiés.

3. Procédé selon la revendication 1, **caractérisé en ce que** la phase aqueuse soutirée est introduite dans le réacteur qui est exploité à température plus basse avec utilisation de catalyseurs au cobalt non modifiés.

4. Procédé selon les revendications 1 à 3, **caractérisé en ce que** l'hydroformylation est réalisée en deux étapes.

5. Procédé selon la revendication 4, **caractérisé en ce que** la température de réaction dans le réacteur qui est exploité à température plus élevée se situe dans la plage de 160 à 220°C et la température de réaction dans le réacteur qui est exploité à température plus basse se situe dans la plage de 120 à 180°C.

6. Procédé selon la revendication 4, **caractérisé en ce que** la température de réaction dans le réacteur qui est exploité à température plus élevée se situe dans la plage de 175 à 195°C et la température de réaction dans le réacteur qui est exploité à température plus basse se situe dans la plage de 150 à 175°C.

7. Procédé selon les revendications 1 à 6, **caractérisé en ce qu'**on hydroformyle des oléfines ou des mélanges d'oléfines comprenant 6 à 24 atomes de carbone.

8. Procédé selon la revendication 7, **caractérisé en ce qu'**on hydroformyle des oléfines ou des mélanges d'oléfines comprenant 8 à 16 atomes de carbone.

9. Procédé selon la revendication 7 ou 8, **caractérisé en ce qu'**on hydroformyle des oligomères de butène.

10. Procédé selon la revendication 9, **caractérisé en ce qu'**on hydroformyle des oligomères préparés à partir de butènes linéaires avec utilisation de catalyseurs en lit fixe au nickel.
